# EUROPEAN PATENT APPLICATION

(11) **EP 4 813 384 A1**
(43) Date of publication of application: **30.09.2026**
(21) Application number: 25789929.4
(22) Date of filing: 16.04.2025
(51) Int. Cl.: A61K 31/423, A61K 45/06, A61P 9/10, A61P 17/02, A61P 13/12, A61P 9/00, A61P 31/00, A61P 29/00, A61P 35/00, A61K 31/44, A61K 31/445, A61K 31/495, A61K 31/4406, A61K 31/4409, A61K 31/439

(54) **COMBINED PHARMACEUTICAL PREPARATION OF TAFAMIDIS OR PHARMACEUTICALLY ACCEPTABLE SALT THEREOF AND PROLINE HYDROXYLASE INHIBITOR**

(30) Priority: 19.04.2024 CN 202410476046
(71) Applicant: Huaya Jiyuan (Shenzhen) Pharmaceutical Co., Ltd., Shenzhen, Guangdong 518122 (CN)
(72) Inventor: XUE, Xiaoqian, Shenzhen, Guangdong 518122 (CN); WEN, Yuanmei, Shenzhen, Guangdong 518122 (CN); ZHONG, Fengxia, Shenzhen, Guangdong 518122 (CN); LI, Chao, Shenzhen, Guangdong 518122 (CN); LI, Shuo, Shenzhen, Guangdong 518122 (CN)
(74) Representative: Metida
(86) International application number: PCT/CN2025/089210
(87) International publication number: WO 2025/218684

(57) **Abstract**

This application relates to the field of biopharmaceutical technology, and in particular, to a pharmaceutical combination preparation of tafamidis or a pharmaceutically acceptable salt thereof in combination with a prolyl hydroxylase inhibitor. The combined use of the tafamidis and the prolyl hydroxylase inhibitor can activate the expression of downstream target genes of HIF-2a, exhibiting a synergistic effect. In a cisplatin-induced renal anemia model, the tafamidis can enhance the therapeutic effect of the prolyl hydroxylase inhibitor in treating renal anemia.

## Description

### TECHNICAL FIELD

This application relates to the field of biopharmaceutical technology, and in particular, to a pharmaceutical combination preparation of tafamidis or a pharmaceutically acceptable salt thereof in combination with a prolyl hydroxylase inhibitor.

### BACKGROUND

The target genes of hypoxia-inducible factor (HIF) are highly diverse and can influence hematopoiesis, angiogenesis, iron ion transport, glucose utilization, resistance-to-oxidative stress, cell differentiation, cell survival and apoptosis, extracellular matrix homeostasis, and tumorigenesis of the body. HIF is a heterodimer formed of α and β subunits. The α subunit is a functional subunit, and is highly sensitive to changes in intracellular oxygen concentration, and is tightly regulated, thereby adjusting HIF activity. The β subunit is a structural subunit, also known as aryl hydrocarbon receptor nuclear translocator (ARNT), and is stably expressed in cells. The mRNA transcription and protein expression levels of the β subunit are not affected by oxygen concentration. Both the α and β subunits of HIF belong to the basic helix-loop-helix (bHLH) transcription factor superfamily. Human HIF-α includes three isoforms: HIF-1α, HIF-2α, and HIF-3α. Among them, HIF-2α exhibits a restricted distribution and plays an important role in the expression and synthesis of erythropoietin (EPO) genes in renal tissue, enhances intestinal iron absorption by upregulating the expression of duodenal cytochrome and divalent metal transporter-1, reduces hepatic antimicrobial peptide expression, and plays a dominant role in iron metabolism. Therefore, HIF-2α plays a specified role in a hypoxic response process.

The HIF-2α subunit belongs to the Per-ARNT-Sim (PAS) subfamily of the bHLH family, and mainly includes an N-terminal bHLH domain (DNA bonding domain, DBD) and two adjacent PASA and PASB domains (ligand bonding domains, LBDs). The C-terminus binds to transcriptional coactivators to regulate transcription of downstream genes. Studies have found that there is a cavity of approximately 290 Å³ within the PASB domain of the HIF-2α subunit. The cavity in combination with a modulator can influence heterodimerization between the HIF-2α subunit and the ARNT subunit, thereby blocking or activating DNA binding and transcription of target genes. Downstream target genes of HIF-2α include vascular endothelial growth factor (VEGF), erythropoietin (EPO), cyclin 1 (Cyclin1), glucose transporter 1 (GLUT1), and the like. Such genes are associated with kidney diseases, renal anemia, cardiovascular diseases, infections, cancers, and the like.

Damage to renal tissue in patients with kidney diseases may lead to insufficient EPO and impaired iron homeostasis, thereby causing renal anemia. Renal anemia not only severely reduces the quality of life of patients, but is also an important factor contributing to the occurrence of cardiovascular diseases and increased mortality. Recombinant human erythropoietin (rHuEPO) or erythropoiesis-stimulating agents (ESAs) can be used to treat renal anemia by increasing hemoglobin levels. However, in clinical trials, a relatively high hemoglobin target level is positively correlated with the risk of cardiovascular adverse events. Currently, an emerging therapy for renal anemia is to pharmacologically inhibit prolyl hydroxylase domain enzymes (PHDs) to stabilize HIF-2 protein, thereby stimulating endogenous EPO production in renal or non-renal tissues. However, excessive upregulation of HIF-1α by the PHD inhibitor promotes inflammatory pathways, accelerates cardiac and renal injury, and consequently increases the risk of pulmonary hypertension and inflammation. The activation of HIF-2α exerts protective effects. Therefore, selective agonists of HIF-2α may offer greater therapeutic advantages.

A typical pathological change in kidney diseases is renal fibrosis. Kyoung HK et al. found that long-term activation of HIF-2α helps suppress renal fibrosis and improve renal function. Yu et al. found that in the early stage of administration of the PHD inhibitor L-mimosine, selective activation of HIF-1α leads to increased expression of fibrotic factor CTGF and phosphorylated Smad, aggravates macrophage infiltration and fibrosis in renal tissue; however, in the middle and late stages, predominant activation of HIF-2α upregulates EPO and VEGF expression and alleviates renal injury. Qu et al. confirmed that knockout of HIF-2α results in more severe renal injury. In summary, in acute and chronic renal injury, excessive activation of HIF-1α may exacerbate renal injury whereas HIF-2α plays a protective role.

Tafamidis (2-(3,5-dichlorophenyl)-1,3-benzoxazole-6-carboxylic acid) is a transthyretin stabilizer originally developed by Pfizer Inc. Vyndamax and Vyndaqel are both capsules for oral administration and contain tafamidis as an active moiety. The U.S. Food and Drug Administration (FDA) has approved Vyndaqel and tafamidis for the treatment of wild-type or hereditary transthyretin-mediated amyloid cardiomyopathy in adults to reduce cardiovascular mortality and cardiovascular-related hospitalization. The European Medicines Agency (EMA) has approved Vyndaqel for the treatment of transthyretin amyloidosis in adult patients with stage I symptomatic polyneuropathy to delay peripheral neurological impairment.

In 2001, studies found that prolyl hydroxylase domain enzymes (PHDs) use O₂ and 2-oxoglutarate (2-OG) as substrates to specifically hydroxylate proline residues on HIF-α, thereby regulating the biological activity of HIF. The catalytic cycle of HIF-PHD is initiated when Fe²⁺ and 2-OG are recruited to the active site of PHD. Subsequently, PHD binds to the proline residues of HIF-α and replaces water molecules with O₂ to complete hydroxylation. Throughout this process, Fe²⁺, O₂, and 2-OG are all essential factors. When cells are under hypoxic conditions, PHD activity is inhibited due to limited O₂ availability, and undegraded HIF-1α and HIF-2α translocate into the nucleus and bind to HIF-β, and act on hypoxia response elements (HREs) to promote the expression of related genes.

Currently, there has been no report on the combined use of tafamidis and a prolyl hydroxylase inhibitor.

### SUMMARY

This application finds that tafamidis acts as an HIF-2α agonist to promote dimerization of HIF-2α/HIF-β, stabilize the bonding between HIF-2α and HIF-β, and increase HIF-2α transcriptional activity in cells, thereby promoting the expression of target genes such as EPO and VEGF and achieving therapeutic effects on diseases associated with HIF-2α activity. Based in this finding, This application discloses a pharmaceutical combination preparation of tafamidis or an analog or salt thereof in combination with a prolyl hydroxylase (PHD) inhibitor. Through combined use in this application, it is found that the combination of the tafamidis (or an analog or salt thereof) and the PHD inhibitor (HIF-PHD) exhibits a synergistic effect, and can inhibit degradation of HIF proteins to treat HIF-2α-mediated diseases.

According to a first aspect, this application provides a pharmaceutical combination preparation that includes tafamidis or a pharmaceutically acceptable salt thereof in combination with a prolyl hydroxylase inhibitor.

In some embodiments, the pharmaceutically acceptable salt of tafamidis is an acid addition salt or a base addition salt.

In some embodiments, the pharmaceutically acceptable salt of tafamidis is at least one selected from the following structures: 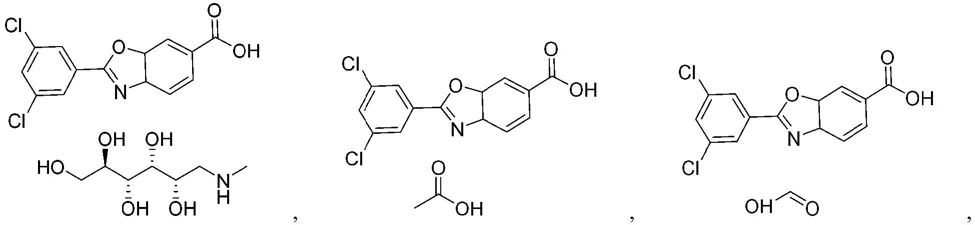 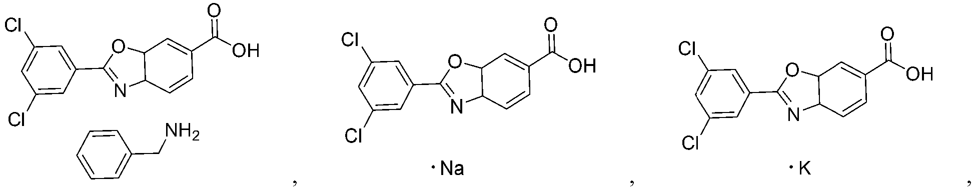 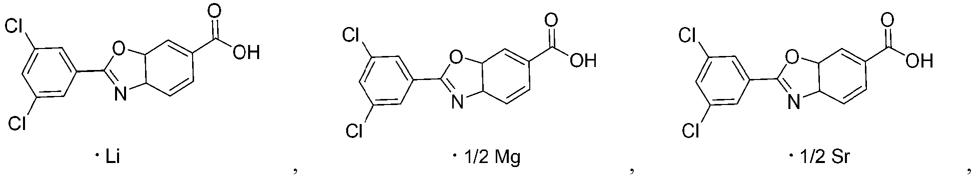 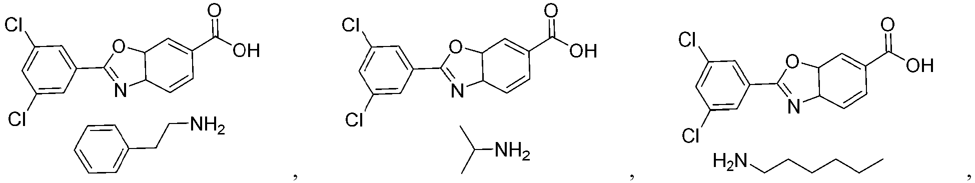 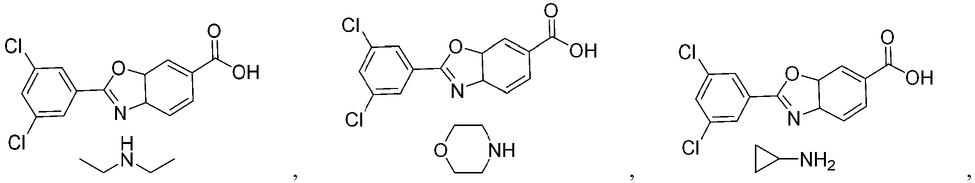 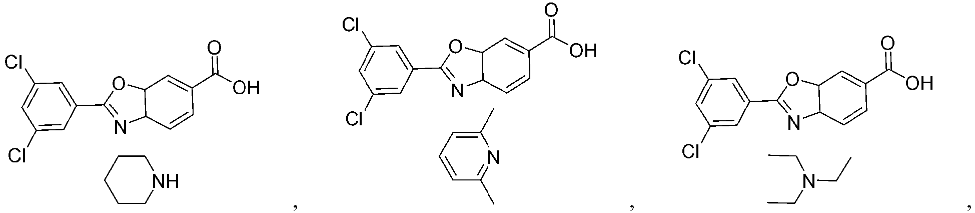 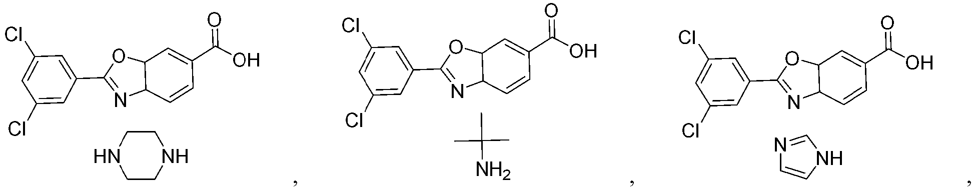 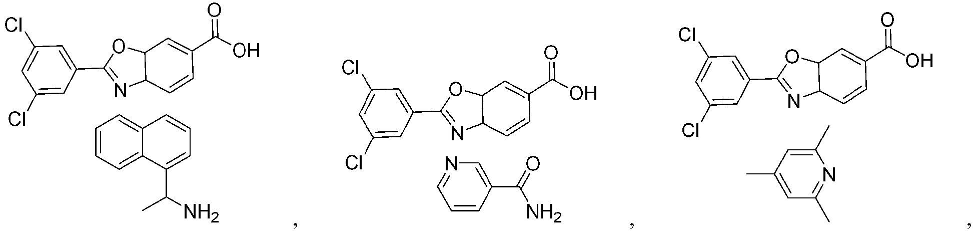 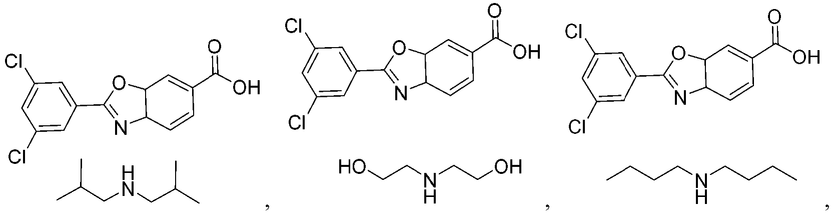 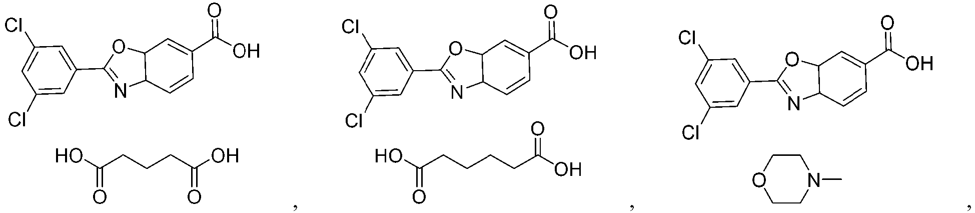 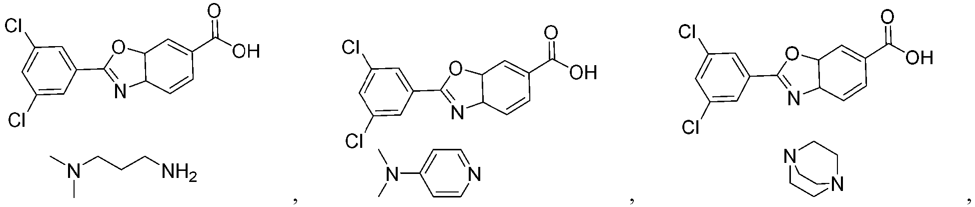 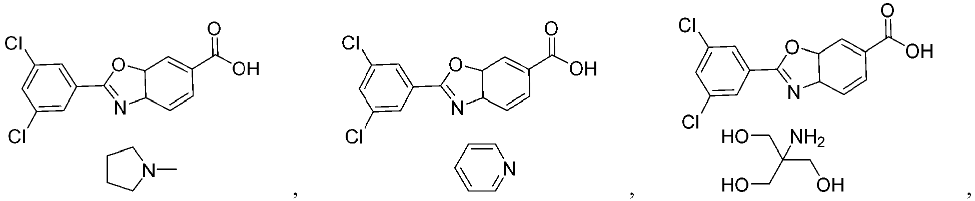 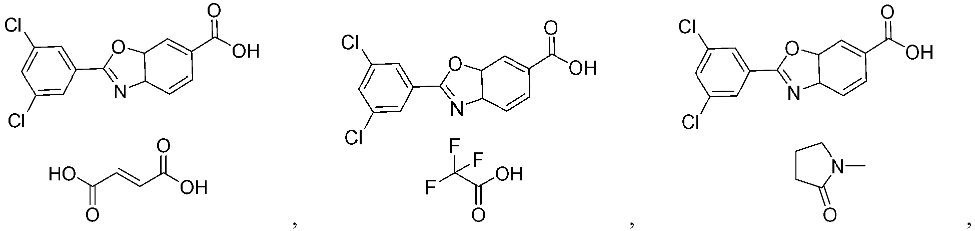 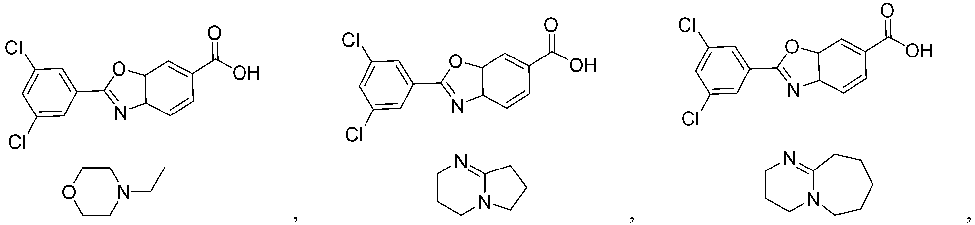 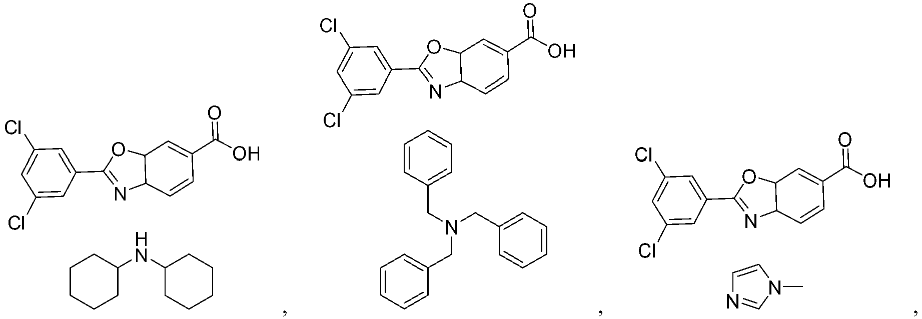 and

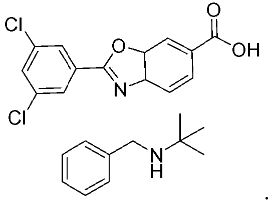

In some embodiments, the prolyl hydroxylase inhibitor includes one or more of roxadustat, daprodustat, vadadustat, enarodustat, or molidustat.

In some embodiments, the pharmaceutical combination preparation further includes a pharmaceutically acceptable excipient.

In some embodiments, in the pharmaceutical combination preparation, a dosage ratio of the tafamidis to the prolyl hydroxylase inhibitor is (1 to 20):10, and preferably 2:1. In some embodiments, in the pharmaceutical combination preparation, a dosage ratio of the tafamidis to the prolyl hydroxylase inhibitor is 1:10, 2:10, 3:10, 4:10, 5:10, 6:10, 7:10, 8:10, 9:10, 10:10, 11:10, 12:10, 13:10, 14:10, 15:10, 16:10, 17:10, 18:10, 19:10, or 20:10.

In some embodiments, the pharmaceutical combination preparation may further include cisplatin.

According to another aspect, this application provides a use of the pharmaceutical combination preparation in manufacturing a drug for preventing, alleviating, or treating a disease associated with HIF-2α activity.

In some embodiments, the disease associated with HIF-2α activity includes hematopoietic disorders, anemia, postoperative surgery-related ischemia and sequelae thereof, postoperative wound healing, chronic kidney diseases, cardiovascular diseases, infections, inflammatory diseases, cancers, impairment of health status occurring during cancer treatment, or sequelae of acute and prolonged cerebral ischemia.

In some embodiments, the disease associated with HIF-2α activity includes: renal anemia; primary anemia; anemia associated with a neoplastic disease; chemotherapy-induced anemia; blood-loss-induced anemia; iron-deficiency-induced anemia; vitamin-deficiency-induced anemia; hypoplastic and aplastic anemia; hemolytic anemia; anemia caused by impaired iron utilization (anemia caused by iron utilization failure) or due to other endocrine disorders (such as hypothyroidism); ischemia and sequelae thereof caused by cardiac interventions using a heart-lung machine (such as bypass surgery and heart valve transplantation), carotid interventions, aortic interventions, and interventions involving opening or penetrating a skull using an instrument; primary glomerulonephritis; hypertensive renal arteriolosclerosis; diabetic nephropathy; secondary glomerulonephritis; tubulointerstitial diseases (chronic pyelonephritis, chronic uric acid nephropathy, obstructive nephropathy, drug-induced nephropathy, and the like); ischemic nephropathy; hereditary nephropathy (polycystic kidney, hereditary nephritis); cardiac insufficiency; coronary heart disease; angina pectoris; myocardial infarction; stroke; arteriosclerosis; primary, pulmonary and malignant hypertension and peripheral arterial occlusive disease; HIV infection; rheumatoid arthritis; diseases of a rheumatic spectrum and other disease forms considered as autoimmune diseases following the use of cytostatics, antibiotics, or radiotherapy; and impairment of health status occurring during pharmacological treatment of such diseases (such as stroke and birth asphyxia).

In some embodiments, the disease associated with HIF-2α activity includes anemia, ischemia, vascular diseases, angina pectoris, myocardial infarction, metabolic disorders, or cancers.

In some embodiments, the disease associated with HIF-2α activity includes renal anemia.

In some embodiments, the tafamidis or a pharmaceutically acceptable salt thereof or the pharmaceutical combination preparation achieves prevention, alleviation, or treatment of diseases associated with HIF-2α activity by upregulating downstream genes regulated by HIF-2α.

In some embodiments, the downstream genes regulated by HIF-2α include vascular endothelial growth factor, erythropoietin, cyclin, and/or glucose transporter.

In some embodiments, the prolyl hydroxylase inhibitor is roxadustat.

### Beneficial effects:

Compared with the prior art, an embodiment of this application achieves at least one of the following beneficial effects:
Through research, this application finds that, during in-vitro experiments, tafamidis can effectively activate HIF-2 transcription, with EC₅₀ being 1.11 µM and the maximum agonistic efficacy being 225%.

In Hep3B cells, roxadustat can increase expression of erythropoietin (EPO) genes. When used in combination with tafamidis that serves as a HIF-2α agonist, the tafamidis can significantly increase EPO gene expression with the increase of concentration.

Through a luciferase reporter gene assay, this application finds that tafamidis and a pharmaceutically acceptable salt thereof, serving as HIF-2α agonists, promote dimerization of HIF-2α and HIF-β and enhance bonding between HIF-2α and HIF-β, thereby significantly enhancing the transcriptional activity of HIF-2α. This activating effect may have potential therapeutic value for treating diseases such as ischemic diseases or anemia.

Through RT-qPCR, it is determined that the tafamidis can upregulate the downstream EPO genes regulated by HIF-2α and, when used in combination with roxadustat, exhibits a synergistic effect. These results indicate that tafamidis and a prolyl hydroxylase drug can be used in combination to activate expression of downstream target genes of HIF-2α. Such activation may have potential therapeutic value for treating diseases such as ischemic diseases or anemia.

In this application, renal anemia symptoms are induced in rats using cisplatin. After being administered for four weeks at 5 mg/kg/day, roxadustat does not produce a significant increase in hemoglobin concentration (HGB) or red blood cell count (RBC) in contrast with the cisplatin group. However, when tafamidis is additionally administered at 10 mg/kg/day, HGB, RBC, and hematocrit (HCT) are all significantly increased. These results indicate that tafamidis, when used in combination with roxadustat, further improves symptoms of renal anemia. In a cisplatin-induced renal anemia model, the tafamidis can enhance the therapeutic effect of the prolyl hydroxylase inhibitor in treating renal anemia, producing an unexpected synergistic enhancement effect.

Through a series of experiments, this application verifies that combined use of tafamidis and roxadustat can synergistically and efficiently prevent and/or treat diseases associated with HIF-2α activity, particularly renal anemia.

### Terminology

Some embodiments of this application are described below in detail, with examples illustrated by the accompanying structural formulas. This application is intended to encompass all substitutions, modifications, and equivalent technical solutions, which are all included within the scope of the protection of this application defined in the claims. Those skilled in the art are aware that a variety of methods and materials similar or equivalent to those mentioned herein are available for implementing this application. This application is in no way limited to the methods and materials described herein. In the event that one or more of the documents, patents, and other similar materials used as a reference herein differ from or contradict this application (including but not limited to the defined terms, term applications, the described technologies, and the like), this application shall prevail.

It is further appreciated that some features of this application, which, for clarity, are described in a plurality of independent embodiments, may also be provided in combination in a single embodiment. Conversely, various features of this application, which, for brevity, are described in a single embodiment, may also be provided separately or in any suitable subcombination.

Unless otherwise specified, all scientific and technical terms used herein bear the same meanings as what are commonly understood by a person skilled in the technical field of this application. All patents and publications related to this application are incorporated herein by reference in their entirety.

In the context of this specification, reference to "one embodiment", "some embodiments", "one or more embodiments", "an embodiment", "specific example", "some examples", and the like means that a particular feature, structure, material, or characteristic described in connection with the embodiment or example is included in at least one embodiment or example of this application. In this embodiment, the appearances of such terms are not necessarily referring to the same embodiment or example. Moreover, the specific features, structures, materials, or characteristics described may be combined in any suitable manner in any one or more embodiments or examples. Furthermore, to the extent that no mutual conflict occurs, those skilled in the art may combine and integrate different embodiments or examples described in this specification, as well as features of different embodiments or examples.

In the following disclosure, all numerical values disclosed herein are approximate, regardless of whether words such as "about" or "approximately" are used. Each numerical value may deviate by 1%, 2%, 5%, 7%, 8%, 10%, 15%, 20%, or the like. Whenever a numerical value N is disclosed, any value of N±1%, N±2%, N±3%, N±5%, N±7%, N±8%, N±10%, N±15%, or N±20% is expressly disclosed, where "±" means plus or minus.

Combination means a fixed combination in a single dosage unit form, or a kit of parts for combination administration, in which the compound disclosed in this application and a combination partner may be administered independently at the same time or may be administered separately within a specified time interval, particularly such that the combination partner exhibits cooperation such as synergy. As used herein, the terms such as "co-administration" or "combination administration" are intended to encompass administering the selected combination partner to a single individual in need thereof (such as a patient), and are intended to include therapeutic regimens in which the substances do not need to be administered by the same means of administration or administered at the same time.

As used herein, the term "drug combination" means a product obtained by mixing or combining at least one active ingredient, and includes both fixed combinations and non-fixed combinations of active ingredients. The term "fixed combination" means that an active ingredient, such as a compound disclosed herein, and a combination partner, are administered simultaneously to a patient in the form of a single entity or dosage. The term "non-fixed combination" means that an active ingredient, such as a compound disclosed herein, and a combination partner, are administered to a patient as separate entities simultaneously, concomitantly, or sequentially with no specific time limitation, and this administration method provides therapeutically effective levels of two compounds in the patient. The latter also applies to cocktail therapy, for example, administration of three or more active ingredients.

### BRIEF DESCRIPTION OF DRAWINGS

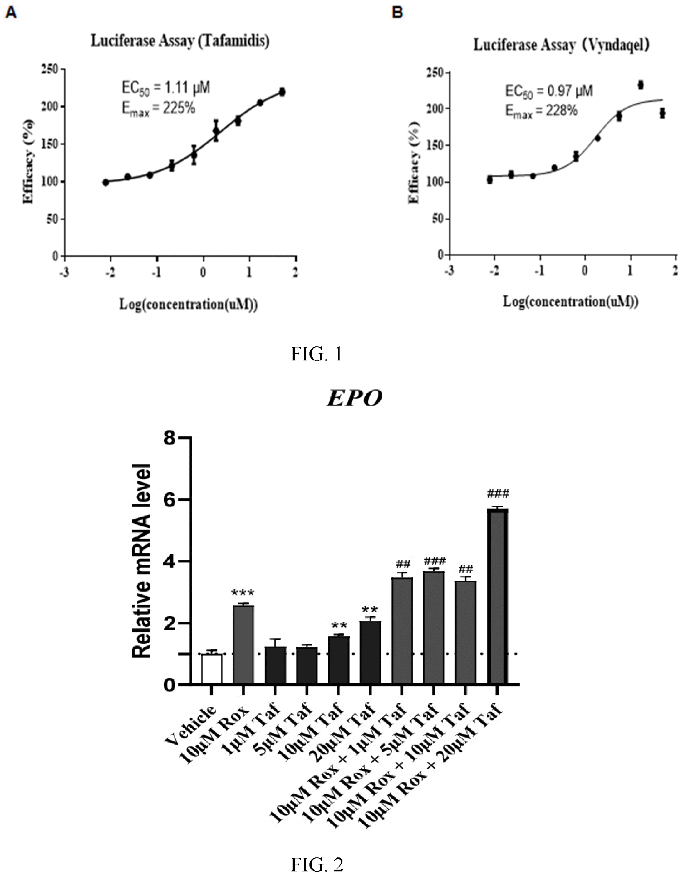

FIG. 1 is a graph illustrating luciferase reporter gene assay results according to Embodiment 1 of this application. In the figure, panel A shows an agonistic activity of tafamidis on HIF-2 protein, and panel B shows agonistic activity of tafamidis meglumine (B) on HIF-2 protein.
FIG. 2 is a graph illustrating real-time quantitative PCR-based assay results according to Embodiment 2 of this application. The results show that, in Embodiment 2 of this application, real-time reverse transcription quantitative polymerase chain reaction (RT-qPCR) detects that tafamidis in combination with roxadustat upregulates transcription of downstream EPO target genes of HIF-2.

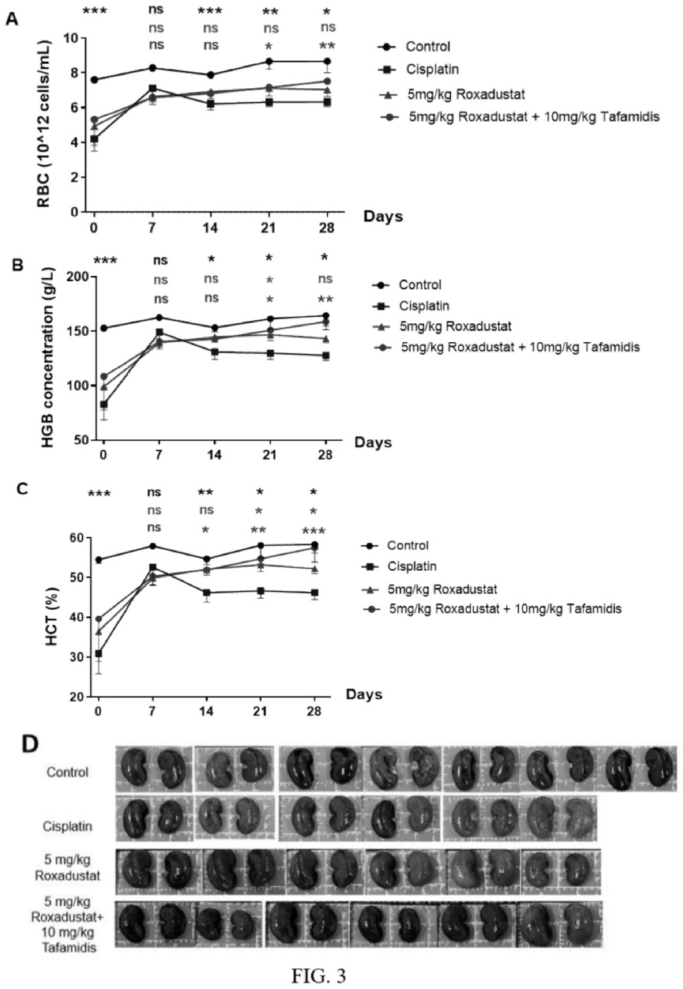

FIG. 3 shows detection results of Embodiment 3. In the figure, panel A is a statistical graph illustrating effects on red blood cell count (RBC) in rats with renal anemia in a cisplatin-induced renal anemia model in different groups; panel B is a statistical graph illustrating effects on hemoglobin levels in rats in a cisplatin-induced renal anemia model in different groups; panel C is a statistical graph illustrating effects on hematocrit (HCT) in a cisplatin-induced renal anemia model in different groups; in panel D, renal tissue images of cisplatin-induced rats in different groups show that, compared with the cisplatin group and the roxadustat monotherapy, the combined use of tafamidis and roxadustat significantly increases renal blood flow.

### DETAILED DESCRIPTION

To make the objectives, technical solutions, and advantages of this application clearer, this application is further described below in detail with reference to embodiments. The specific embodiments described herein are merely intended to explain this application without constituting any limitation on this application. In addition, in the following description, descriptions of well-known structures and technologies are omitted to avoid unnecessary confusion about the concepts of this application. Such structures and technologies are also described in many publications.

All reagents used in this application may be purchased from the market or prepared by using the method described in this application.

### Embodiment 1: Luciferase reporter gene assay

Experimental principles: 786-O is a renal carcinoma cell line that predominantly expresses HIF-2α and lacks functional HIF-1α. This characteristic makes 786-O cells an ideal model for studying HIF-2α transcriptional activity and related signaling pathways. In the luciferase reporter gene assay, a 786-O monoclonal cell line that stably expresses hypoxia response element (HRE)-driven luciferase (Luc) activity is obtained through puromycin selection. The HIF-2α agonist promotes dimerization of HIF-2α and HIF-β, and activates an HRE promoter. By measuring fluorescence intensity, luciferase activity can be determined, thereby assessing the ability of the HIF-2α agonist to promote transcription.

Experimental method: A total of 6000 stably transfected 786-O cells stably containing HRE and Luc sequences are seeded in each well of a 96-well plate, and 100 µL of RPMI-1640 culture medium containing 10% fetal bovine serum is added to each well. After 24 hours, a compound at a corresponding concentration is added to each well. A blank group is set as a control. Only a DMSO solvent is added in the well of the blank group. For initial compound screening, both tafamidis and tafamidis meglumine are tested at two concentrations: 2 µM and 20 µM. For concentration-gradient determination, each concentration is tested in triplicate. After co-incubation for 24 hours, the culture medium is discarded, and 20 µL of firefly luciferase reporter gene cell lysis buffer (RG126M, Beyotime) is added to each well. The 96-well plate is shaken on a microplate shaker for 10 minutes. After shaking, 10 µL of lysate is transferred into a white opaque plate, and then 10 µL of Steady-Lumi^{™} firefly luciferase assay reagent (RG058S, Beyotime) is added to each well. Finally, luminescence is measured using a microplate reader (Agilent Synergy Neo2). E₂₀ represents the effect of the compound at a 20 µM concentration, and is calculated as: E₂₀ = fluorescence value of the 20 µM well ÷ fluorescence value of the blank control × 100%. E₂ represents the effect of the compound at a 2 µM concentration, and is calculated as: E₂ = fluorescence value of the 2 µM well ÷ fluorescence value of the blank control × 100%. EC₅₀ is the compound concentration at which the agonistic efficacy is increased by 50% relative to the blank control. The agonistic efficacy of a compound is determined by calculating the concentration (EC₅₀) required to achieve a 50% increase in the fluorescence activity relative to the blank control and a maximum activation percentage (Eₘₐₓ). The maximum activation percentage (Eₘₐₓ) of an agonist refers to a ratio, expressed as a percentage, of the fluorescence value corresponding to the maximal effect (observed maximal response) induced when the agonist concentration reaches receptor-binding saturation in a particular receptor system, to the fluorescence value of the receptor system in the absence of agonist. In this experiment, Vyndaqel is directly used in place of tafamidis meglumine.

The test results show that the agonistic efficacy at a 20 µM concentration tafamidis is 207% while the agonistic efficacy at a 2 µM concentration is 142%, indicating that the agonistic efficacy of tafamidis increases with the increase of the concentration, exhibiting a clear dose dependence. As measured, EC₅₀ of tafamidis is 1.11 µM, and Eₘₐₓ is 225% (FIG. 1A), indicating that tafamidis exhibits strong agonistic activity and favorable efficacy.

The agonistic efficacy of tafamidis meglumine at a 20 µM concentration is 223% while the agonistic efficacy at a 2 µM concentration is 122%, indicating that the agonistic efficacy of the tafamidis meglumine increases with the increase of concentration, exhibiting a clear dose dependence. As measured, EC₅₀ of the tafamidis meglumine is 0.97 µM, and Eₘₐₓ is 228% (FIG. 1B), indicating that the tafamidis meglumine also exhibits strong agonistic activity and favorable efficacy.

As can be seen, tafamidis and a pharmaceutically acceptable salt thereof, serving as HIF-2α agonists, promote dimerization of HIF-2α and HIF-β and enhance bonding between HIF-2α and HIF-β, thereby promoting the transcription of HIF-2α downstream genes.

### Embodiment 2: Real-time reverse transcription quantitative polymerase chain reaction (qRT-PCR) assay

Experimental method: HepG2 hepatocellular carcinoma cells are seeded into a 12-well plate. After 24 hours, 10 µM roxadustat, 1 µM tafamidis, 5 µM tafamidis, 10 µM tafamidis, and 20 µM tafamidis, a combination of 10 µM roxadustat and 1 µM tafamidis, a combination of 10 µM roxadustat and 5 µM tafamidis, a combination of 10 µM roxadustat and 10 µM tafamidis, and a combination of 10 µM roxadustat and 20 µM tafamidis, are added separately, and the cells are co-incubated with the compounds for 24 hours. No treatment is applied to the vehicle group. RNA is extracted using a TRIZOL reagent. cDNA transcription is performed using an All-in-one^{™} First-Strand cDNA Synthesis Kit (for specific operating procedures, see the manufacturer's instructions). A SYBR reagent is used for fluorescence signal detection, and GAPDH is used as an internal control. The qRT-PCR primers are as follows:
GAPDH_fwd, GCACCGTCAAGGCTGAGAAC;
GAPDH_rev, TGGTGAAGACGCCAGTGGA;
EPO_fwd, AACAATCACTGCTGACACTT;
EPO_rev, AGAGTTGCTCTCTGGACAGT.

The test results are shown in FIG. 2: relative to the vehicle (control group), monotherapy with 10 µM roxadustat upregulates erythropoietin (EPO) gene expression by 1.5-fold, while monotherapy with 20 µM tafamidis upregulates EPO gene expression by 1-fold. Different concentrations of tafamidis show an upregulatory effect on EPO gene expression, indicating that tafamidis can effectively activate the expression of HIF-2α downstream target genes. When tafamidis at different concentrations is used in combination with 10 µM roxadustat, the combination of 10 µM roxadustat and 20 µM tafamidis upregulates EPO gene expression by 4.7-fold, exhibiting a synergistic effect. These results indicate that tafamidis and a prolyl hydroxylase drug can be used in combination to activate expression of downstream target genes of HIF-2α. Such activation may have potential therapeutic value for treating diseases such as ischemic diseases or anemia. Statistical analysis is performed using SPSS software. Compared with the no-treatment group: *P < 0.05, **P < 0.01, ***P < 0.001. Compared with the 10 µM roxadustat group: #P < 0.05, ##P < 0.01, and ###P < 0.001.

### Embodiment 3 Cisplatin-induced renal anemia rat model

Male Sprague-Dawley (SD) rats are allowed a one-week acclimation period, after which cisplatin (6 mg/kg) is administered through tail vein injection. A second injection is given one week later. Two weeks after the first administration, successful establishment of a renal disease model is confirmed by measuring serum creatinine and urea levels. The successfully modeled rats are randomly divided into three groups: cisplatin group (n = 6), 5 mg/kg roxadustat group (n = 6), and 5 mg/kg roxadustat + 10 mg/kg tafamidis group (n = 6), with continuous administration for 4 weeks. A solvent is administered to the normal control group (n = 7) and the cisplatin group based on the same drug administration regimen. After drug administration, blood analysis is performed once per week using a Mindray automated hematology analyzer (BC-5150). After sample collection, the rats are euthanized. Statistical significance is determined by one-way analysis of variance (ANOVA), and then a Dunnett's test is performed for comparison with the cisplatin group (*P < 0.05, **P < 0.01, ***P < 0.001).

Hematological analysis at Week 4 is shown in Table 1 below:

**Table 1**

| | Cisplatin group | 5 mg/kg roxadustat group | 5 mg/kg roxadustat + 10 mg/kg tafamidis group |
|---|---|---|---|
| Red blood cell count (FIG. 3A) (unit: ×10¹² cells/L) | 6.33 ± 0.27 | 7.03 ± 0.40 | 7.52 ± 0.13 |
| Hemoglobin concentration (FIG. 3B) (unit: g/L) | 127 ± 4.7 | 143.3 ± 4.1 | 158.5 ± 3.8 |
| Hematocrit (FIG. 3C) (unit: %) | 46.2 ± 1.8 | 52.2 ± 1.3 | 57.4 ± 1.3 |

Results analysis: The results in Table 1 and FIG. 3A to FIG. 3C indicate that the tafamidis, when used in combination with roxadustat, further enhances the activity of the roxadustat.

Gross observation of kidney tissues (FIG. 3D) shows that the kidneys in the normal control group exhibit a healthy red color, while those in the cisplatin group show obvious yellow discoloration. In contrast, in the 5 mg/kg roxadustat group, the kidneys of 3 rats appear red, while in the 5 mg/kg roxadustat + 10 mg/kg tafamidis group, the kidneys of 5 rats appear red. These results indicate that tafamidis, when used in combination with roxadustat, further improves symptoms of renal anemia.

Mechanistically, roxadustat stabilizes the HIF-2α protein by inhibiting the activity of prolyl hydroxylases. To regulate the transcriptional activity of downstream genes, HIF-2α needs to be dimerized with HIF-β to form a stable dimer. In Embodiment 1, it is demonstrated that the tafamidis serving as an HIF-2α agonist promotes dimerization of HIF-2α and HIF-β, enhances the bonding between HIF-2α and HIF-β, thereby promoting transcription of genes of HIF-2α. In Embodiment 2, the combined use of tafamidis and a prolyl hydroxylase inhibitor enhances transcription of the downstream EPO genes of HIF-2α. In Embodiment 3, the combined use of tafamidis and a prolyl hydroxylase inhibitor roxadustat enhances the pharmacological activity of roxadustat. This study is of significant importance for patients treated with roxadustat alone.

The methods disclosed in this application have been described with reference to preferred embodiments. Evidently, those skilled in the art can implement and apply the technical solutions of this application by modifying or appropriately varying and combining the methods and applications described herein without departing from the subject-matter, essence, and scope of this application. Those skilled in the art may implement the technical solutions by appropriately modifying some process parameters with reference to the teachings hereof. It is particularly important to note that all similar replacements and modifications are apparent to those skilled in the art and are deemed to fall within the scope of protection of this application.

## Claims

1. A pharmaceutical combination preparation, comprising tafamidis or a pharmaceutically acceptable salt thereof in combination with a prolyl hydroxylase inhibitor.

2. The pharmaceutical combination preparation according to claim 1, **characterized in that** the pharmaceutically acceptable salt of tafamidis is an acid addition salt or a base addition salt.

3. The pharmaceutical combination preparation according to claim 1, **characterized in that** the pharmaceutically acceptable salt of tafamidis is at least one selected from the following structures: 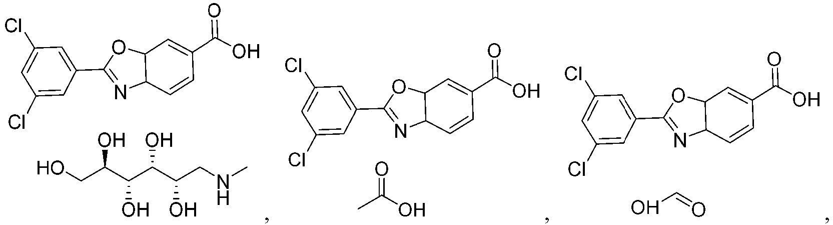 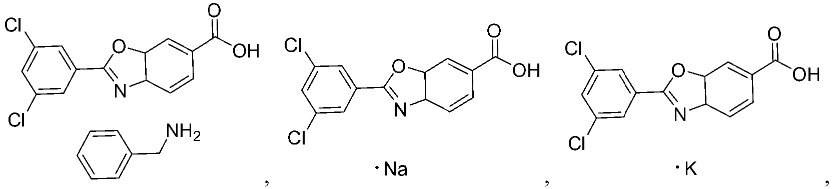 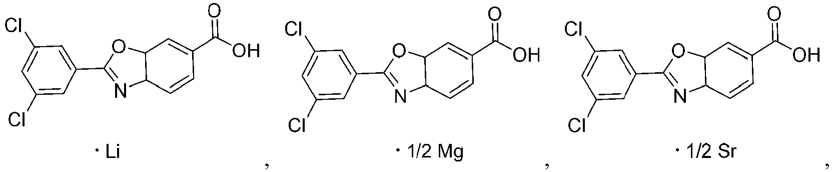 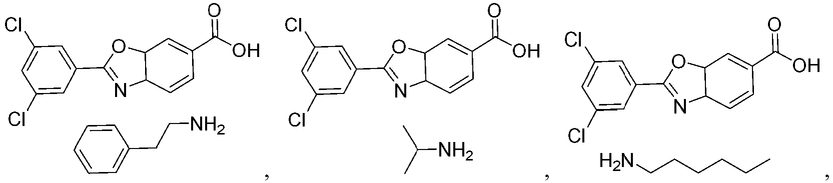 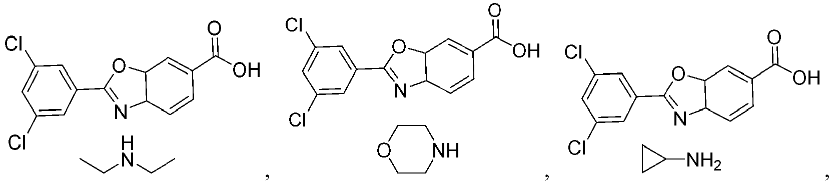 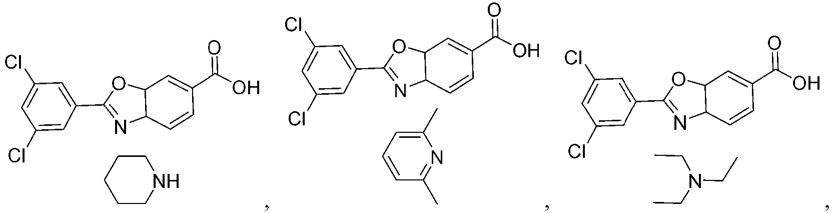 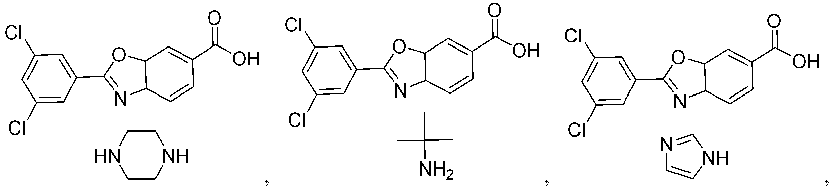 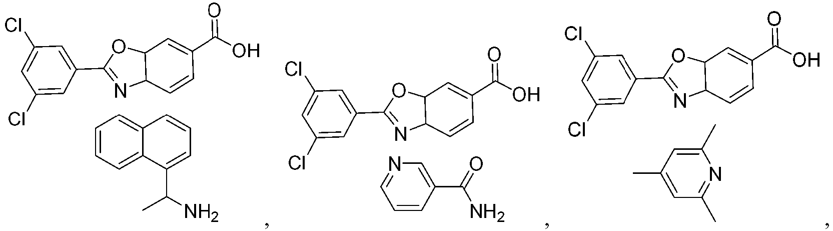 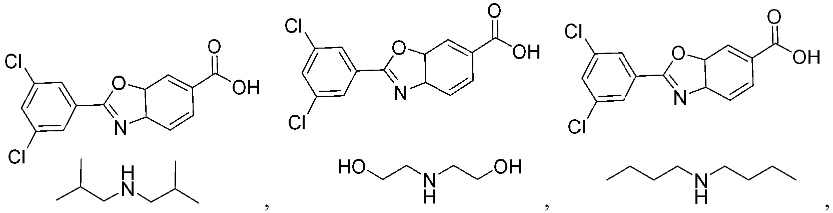 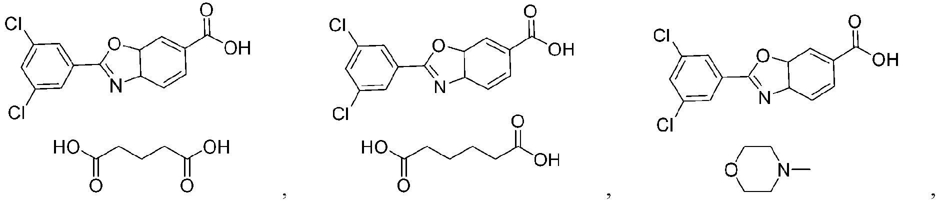 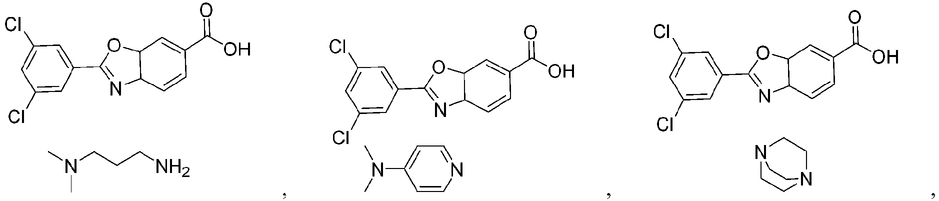 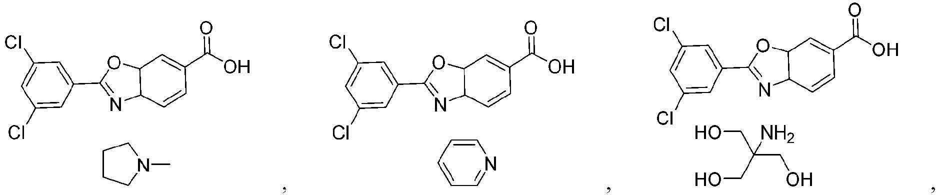 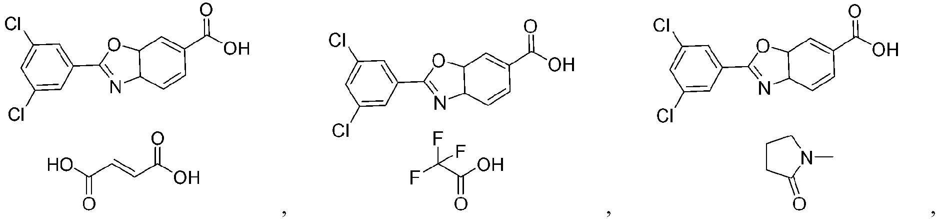 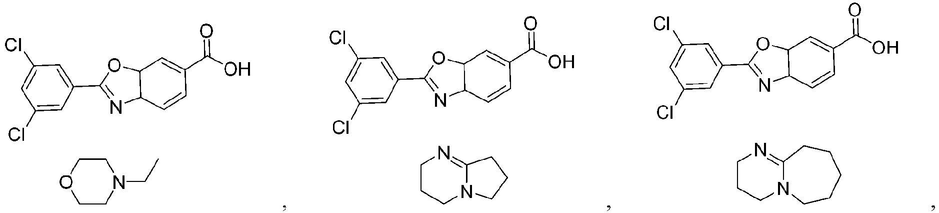 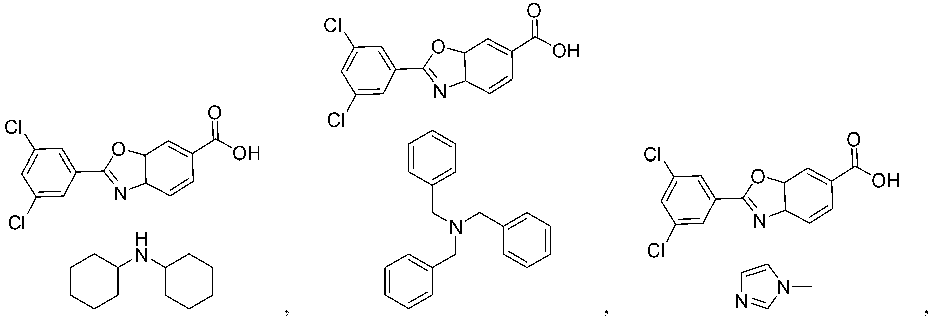 or

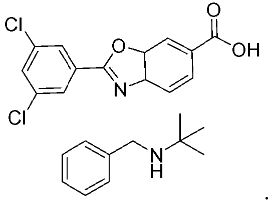

4. The pharmaceutical combination preparation according to claim 1, **characterized in that** the prolyl hydroxylase inhibitor comprises one or more of roxadustat, daprodustat, vadadustat, enarodustat, or molidustat.

5. The pharmaceutical combination preparation according to claim 1, **characterized in that** the pharmaceutical combination preparation further comprises a pharmaceutically acceptable excipient.

6. The pharmaceutical combination preparation according to claim 1, **characterized in that** in the pharmaceutical combination preparation, a dosage ratio of the tafamidis to the prolyl hydroxylase inhibitor is (1 to 20):10.

7. A use of the pharmaceutical combination preparation according to any one of claims 1 to 6 in manufacturing a drug for preventing, alleviating, or treating a disease associated with HIF-2α activity.

8. The use according to claim 7, **characterized in that** the disease associated with HIF-2α activity comprises hematopoietic disorders, anemia, postoperative surgery-related ischemia and sequelae thereof, postoperative wound healing, chronic kidney diseases, cardiovascular diseases, infections, inflammatory diseases, cancers, impairment of health status occurring during cancer treatment, or sequelae of acute and prolonged cerebral ischemia;
or, the disease associated with HIF-2α activity comprises: renal anemia; primary anemia; anemia associated with a neoplastic disease; chemotherapy-induced anemia; blood-loss-induced anemia; iron-deficiency-induced anemia; vitamin-deficiency-induced anemia; hypoplastic and aplastic anemia; hemolytic anemia; anemia caused by impaired iron utilization (anemia caused by iron utilization failure) or due to other endocrine disorders (such as hypothyroidism); ischemia and sequelae thereof caused by cardiac interventions using a heart-lung machine (such as bypass surgery and heart valve transplantation), carotid interventions, aortic interventions, and interventions involving opening or penetrating a skull using an instrument; primary glomerulonephritis; hypertensive renal arteriolosclerosis; diabetic nephropathy; secondary glomerulonephritis; tubulointerstitial diseases (chronic pyelonephritis, chronic uric acid nephropathy, obstructive nephropathy, drug-induced nephropathy, and the like); ischemic nephropathy; hereditary nephropathy (polycystic kidney, hereditary nephritis); cardiac insufficiency; coronary heart disease; angina pectoris; myocardial infarction; stroke; arteriosclerosis; primary, pulmonary and malignant hypertension and peripheral arterial occlusive disease; HIV infection; rheumatoid arthritis; diseases of a rheumatic spectrum and other disease forms considered as autoimmune diseases following the use of cytostatics, antibiotics, or radiotherapy; and impairment of health status occurring during pharmacological treatment of such diseases (such as stroke and birth asphyxia);
or, the disease associated with HIF-2α activity comprises anemia, ischemia, vascular diseases, angina pectoris, myocardial infarction, metabolic disorders, or cancers; or, the disease associated with HIF-2α activity comprises renal anemia and/or nephropathy.

9. The use according to claim 7, **characterized in that**
the pharmaceutical combination preparation according to any one of claims 1 to 6 achieves prevention, alleviation, or treatment of diseases associated with HIF-2α activity by upregulating downstream genes regulated by HIF-2α.

10. The use according to claim 9, **characterized in that**
the downstream genes regulated by HIF-2α comprise vascular endothelial growth factor, erythropoietin, cyclin, and/or glucose transporter.
